# EUROPEAN PATENT SPECIFICATION

(11) **EP 1 516 581 B1**
(45) Date of publication and mention of the grant of the patent: **13.07.2011**
(21) Application number: 04254377.7
(22) Date of filing: 22.07.2004
(51) Int. Cl.: A61B 5/0484, A61B 5/0478

(54) **A combined passive and active neuromonitoring device**
Gerät für kombiniertes passives und aktives Neuromonitoring
Appareil pour le combination de neuromonitorage passive et active

(30) Priority: 17.09.2003 US 667237
(43) Date of publication of application: 23.03.2005
(73) Proprietor: INSTRUMENTARIUM CORPORATION, 00031 Datex-Ohmeda (FI)
(72) Inventor: Merilainen, Pekka, 00660 Helsinki (FI); Viertio-Oja, Hanna E., 02760 Espoo (FI); Huiku, Matti, 02760 Espoo (FI)
(74) Representative: Charlton, Peter John

(56) References cited:
- EP-A- 1 238 628
- WO-A-01/37724
- WO-A-02/100267
- WO-A-03/057030
- WO-A-2004/071289
- US-A- 5 954 667
- US-A- 6 067 467
- US-A- 6 167 298
- US-B1- 6 394 953
- CAPITANIO L ET AL: "ON-LINE ANALYSIS OF AEP AND EEG FOR MONITORING DEPTH OF ANAESTHESIA" METHODS OF INFORMATION IN MEDICINE, XX, XX, vol. 36, no. 4/5, December 1997 (1997-12), pages 311-314, XP000866409 ISSN: 0026-1270

## Description

The present invention relates to a sensor for obtaining electrophysiological biopotential signals, such as EEG or EMG signals. More specifically, the present invention relates to a unitary sensor that utilizes passive neuromonitoring during deep levels of sedation and active neuromonitoring during low levels of sedation and consciousness.

### BACKGROUND OF THE INVENTION

Neuromonitoring is a subfield of clinical patient monitoring focused on measuring various aspects of brain function and changes in the brain function caused by drugs commonly used to induce and maintain anesthesia in the operation room or sedation in patients under critical or intensive care.

Electroencephalography (EEG) is a well established method for assessing brain activity by recording and analyzing the weak biosignals generated in the cortex of the brain with electrodes attached on the skin of the skull surface. EEG has been in wide use for decades in basic research of the neural systems of the brain as well as in the clinical diagnosis of various neurophysiological diseases and disorders.

One of the special applications to which a significant amount of attention has been devoted during the past few years is use of the processed EEG signals for the objective quantification of the brain function for the purpose of determining the level of consciousness. The basic idea is to automatically detect if the subject or patient is asleep or awake. Specifically, this has become an issue, both scientifically and commercially, in the context of measuring the depth of anesthesia during surgery. The modem anesthesia delivery process uses sophisticated techniques combining the balanced use of several drugs for maintaining adequate hypnosis, analgesia, muscle relaxation, suppression of the autonomic nervous system and blockade of the neuromuscular junction.

The need for reliable monitoring of the adequacy of anesthesia is based on both safety and economy related aspects. Too light an anesthesia, or in the worst case the patient waking up in the middle of the operation, may cause a traumatic experience both for the patient and for the anesthesia personnel. Too deep an anesthesia may cause increased perioperative costs through extra use of drugs and time and as well as lengthening the time required for the post-operative care. Too deep of sedation of the ICU patients may also cause complications and prolong the expensive usage time for the intensive care theater.

In certain contexts, the EEG method is also called *passive* neuromonitoring because it is essentially recording the spontaneous electrical activity of the brain. Another measurement mode is labeled *active* neuromonitoring, in which the responses of the brain to specific external stimuli are under investigation. A typical stimulus is a short click of sound, which in turn generates a weak EEG response signals in various parts of the brain typically within a few tens of milliseconds after the click. The EEG responses are called Auditory Evoked Potentials (AEP) which are a subset of Event Related Potentials (ERP). The stimulus in the ERP method can, for example, be a more complex sound burst or even a visual stimulus such as a light flash. The AEP are so weak compared to the background EEG that the stimulus and response have to be repeated at least 200 times to be able to extract the shape of the response curve in a synchronized integration process, in which the background signal of more or less random nature will be cancelled.

An AEP response is an attenuating oscillation wave of a few swings up and down. All the positive and negative peaks have standardized notations and the parameters of interest are the amplitudes of the wave peaks and their delay from the stimulus time, which is referred to as latency in this context. Qualitatively speaking, when the awareness of the subject decreases, the amplitude of peaks decrease and their latency increases.

The AEP's can be divided to various groups depending on the latency. The brain stem responses appear during the first 10 milliseconds, the early cortical responses from 10 to 80 ms (middle latency AEP's, MLAEP), and the late cortical responses appear from 80 to 1000 ms (long latency AEP's, LLAEP). So, in simple terms, the brain reacts weaker and slower to external stimuli when the awareness is reduced, such as during anesthesia.

During the past few years, several commercial devices for measuring the level of consciousness and/or awareness in a clinical set-up during anesthesia that utilize either passive or active neuromonitoring have become available. Passive devices that are based on a processed one-channel EEG-signal have been introduced by Aspect Medical (Bispectral Index) and Datex-Ohmeda (Entropy Index). An active monitoring device has been introduced by Danmeter (AAI™) based on MLAEP with a special auto regression based algorithm to extract the key features of the signal, and has been marketed for monitoring consciousness and awareness of the patient during anesthesia.

The key difference in the performance capability between these two methods is that the modern passive monitoring devices can reliably cover the whole range of consciousness from awake and fully alert, through the threshold of falling asleep, down to the deepest sedation characterized by EEG suppression where the EEG signal is reduced to a straight line. Active monitoring devices, on the other hand, are able to accurately differentiate the decreasing levels of awareness only down to the level of falling asleep. After the patient is asleep, the signal disappears and no information is obtained regarding deeper levels of sedation.

Based on above, passive monitoring methods are generally more useful when the whole range of the consciousness must be monitored, such as in measuring the adequacy of anesthesia for keeping the patient safely on an optimal level of sleep to prevent inadvertent waking up in the middle of an operation. However, in many cases patients in intensive care units (ICU) are kept conscious and under light or moderate sedation and there is a need to quantify the level of sedation objectively. The current practice is to rely upon a subjective assessment carried out by the care personnel by verbal or tactile prompting with help of standardized scoring tables. However, it is quite common that ICU patients are kept in deep anesthetic levels of sedation during the first days of intensive care. During this period, any measurement method should also work reliably at the deeper levels as well, which is possible only by using the passive method.

Thus, a need exists for a method of neuromonitoring that can utilize passive monitoring methods for monitoring EEG during deeper levels of sedation while utilizing active monitoring during periods in which a patient is kept conscious and under light or moderate sedation. Further, a need exists for such monitoring system that utilizes a common sensing element to carry out both active and passive monitoring of EEG signals.

US-A-6167298 discloses devices for monitoring and maintaining an alert state of consciousness in a subject wearing the device. The device can be a unitary sensor including at least three electrodes on a base strip. An alert mental state is maintained through monitoring of brain wave patterns to detect if a transition from an alert to a non-alert mental state is about to occur, or has occurred. If so, a stimulus, e.g., an audible tone, is provided until such time as an alert mental state, as assessed by brain wave activity, is restored. Also disclosed are methods for maintaining an alert mental state, as well applications for such devices and methods.

WO-A-03/57030 discloses a physiological sensor combination which has a flexible substrate configured to attach to a tissue site. Multiple sensors are disposed on the substrate, which generate physiological signals. Each of the signals is responsive to a different physiological parameter. Conductors are carried on the substrate and routed between the sensors and at least one connector. The connector is configured to communicate the physiological signals to at least one monitor, which derives measurements of the parameters.

US-B-6394953 discloses an array of electrodes which is constructed to allow the user to easily adjust to the correct size of the patient's head. The array is self adhesive, pre-gelled and disposable. The array fits easily over the temple and forehead areas where EEG signals can be acquired by specially designed monitors for purposes of monitoring a number of bodily phenomena, including but not limited to, depth of anesthesia, and/or ischemia, and burst suppression. The array is connected to the monitor via a tab connector that is integral to the disposable device. The tab connector is insertible into a reusable connector that is part of a monitoring system.

### SUMMARY OF THE INVENTION

A unitary sensor constructed in accordance with the present invention comprises a unitary sensor for detecting biopotential signals on the skin of a patient, the sensor comprising a base strip including at least three electrodes spaced along then length of the base strip, each of the sensors being operable to detect biopotential signals on the skin of the patient; and characterised by an extension strip integrally formed with the base strip and extending from the base strip to a distal end, the extension strip including an acoustic emitter attached to the distal end, and the base strip being configured to be attached to the forehead of the patient and the extension strip extends from the base strip such that the acoustic emitter is positionable in an ear of the patient when the base strip is positioned on the forehead of the patient.

The acoustic emitter is coupled, through a pair of leads, to the control unit such that the acoustic emitter can be utilized to create acoustic stimuli during active monitoring. Preferably, the acoustic emitter is surrounded by a soft, flexible plug such that the acoustic emitter can be received within the patient's ear. Thus, the single unitary sensor constructed in accordance with the invention can be utilized for both active monitoring using acoustic stimuli and passive monitoring.

The present invention can be used in a combined neuromonitoring method and device to measure biopotential signals in sedated ICU patients over the whole range of sedation from being fully alert to the complete suppression of EEG. The present invention utilizes an integrated sensor combining an acoustic emitter and multiple EEG electrodes on a single, lightweight disposable component. The method utilizes active monitoring during light sedation and passive monitoring during deep sedation with adequate intelligence to handle transition from one mode to another.

Briefly, the above device can incorporate a control unit that is coupled to both an active monitoring module and a passive monitoring module. The passive monitoring module is utilized to record the spontaneous electrical activity of the brain by monitoring EEG signals. The passive monitoring module is particularly desirable in monitoring the level of sedation when a patient is deeply sedated.

The active monitoring module of the system utilizes external stimuli to induce a weak EEG response signal in various parts of the brain. This response, referred to as auditory evoked potentials (AEP), is particularly desirable in determining the level of sedation when the patient is awake or only moderately sedated. The control unit of the present invention selects between the active and passive monitoring modules depending upon the sensed level of sedation. As an illustrative example, the control unit utilizes active monitoring when the patient is at Ramsay levels R1-R3 and utilizes passive monitoring when the patient is at Ramsay levels R4-R6. The control unit displays the output of only the active or passive module depending upon the sensed level of sedation.

Preferably, the electrodes are also configured to detect AEP signals when the system is used in an active monitoring mode. Thus, the single base strip can be utilized during both active and passive monitoring situations.

Various other features, objects and advantages of the invention will be made apparent from the following description taken together with the drawings.

### BRIEF DESCRIPTION OF THE DRAWING

The drawings illustrate the best mode presently contemplated of carrying out the invention.

In the drawings:
Fig. 1 is a general schematic illustrating the method of utilizing the active and passive patient monitoring in accordance with the present invention.
Fig. 2 is a graphic illustration of a typical AEP signal during a level of sedation;
Fig. 3 is a general view of the unitary, multi-electrode biopotential signal sensor and acoustic emitter of the present invention in use with a patient; and
Fig. 4 is a plan view of the sensor of the present invention showing the side of the sensor applied to the skin of the patient.

### DETAILED DESCRIPTION OF THE INVENTION

In order to understand the present invention and the method of selecting between active and passive neuromonitoring, the Ramsay Score (RS), which is used to signify six different levels of sedation, will be used throughout the foregoing description. Listed below is a chart illustrating the six levels of the Ramsay Score and the clinical response of the patient for each level:

| Sedation Score | Clinical Response of the Patient |
|---|---|
| RS 1 | Awake/agitated |
| RS 2 | Lightly sedated |
| RS 3 | Moderately sedated |
| RS 4 | Deeply sedated, responds to nonpainful stimuli |
| RS 5 | Deeply sedated, responds to painful stimuli |
| RS 6 | Deeply sedated, unresponsive to painful stimuli |

When using passive methods for determining the level of sedation within a patient, problems arise during Ramsay levels RS1 to RS3 when utilizing EEG measurements taken from the forehead of the patient. These problems are primarily related to the artifacts introduced into the measured signals that are generated by eye movement and the electrical activity of the frontal muscle (EMG). When the patient is in the Ramsay levels RS to RS3, active monitoring includes inherent benefits, since during levels RS to RS3, the low frequencies introduced into the EEG measurement related to eye artifacts can be filtered out and the contribution of the EMG is also removed by averaging the signal over a large number of impulses. Although active monitoring in Ramsay levels RS1 to RS3 has proven to be very useful, active monitoring by utilizing sound stimuli is useless in subjects with impaired hearing.

One type of active monitoring using sound stimuli is taught and described in published PCT patent application WO 01/74248 and is included in the AAI™ monitor available from Danmeter. In active monitoring using auditory stimulation, an auditory stimulus, such as a click, is supplied to the patient, which in turn generates a weak EEG response in various parts of the brain typically within a few tenths of a millisecond after the click. The EEG response is called an auditory evoked potential (AEP).

The criteria for continuing to use active monitoring is typically defined as a minimum amplitude of the relevant AEP peak that is reliably detectable. When the amplitude of the peak gets smaller than an acceptable threshold, which typically occurs between Ramsay levels RS4 and RS5, the active monitoring system no longer becomes reliable.

One illustrative example of the passive monitoring is described in published PCT patent application WO 02/32305, which is typically carried out to provide an indication of the depth of anesthesia that a patient is experiencing. As described above, the passive monitoring module is particularly desirable for determining the depth of sedation when a patient is in the Ramsay levels R4 to R6, while the active monitoring module is particularly desirable in determining the level of sedation when the patient is in Ramsay levels R1 to R3.

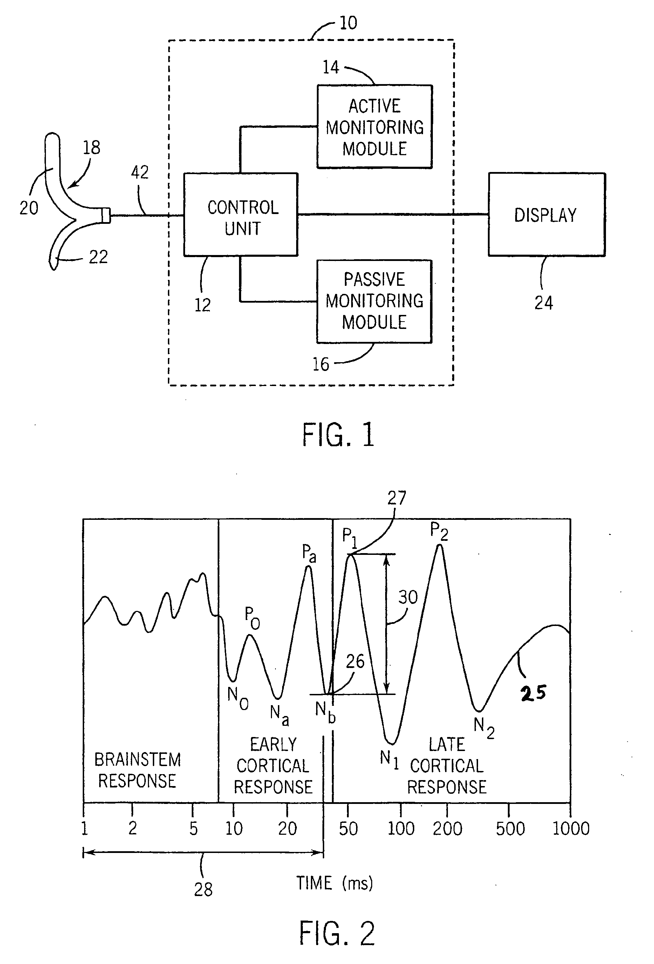

Referring now to Fig. 1, thereshown is a general schematic illustration of a system that can be used with the unitary sensor of the present invention. As illustrated, the present invention is directed to a combined passive and active neuromonitoring system 10. The system 10 generally includes a central control unit 12 connected to both an active monitoring module 14 and a passive monitoring module 16. The control unit 12 selects between the active monitoring module 14 and the passive monitoring module 16 depending upon the Ramsay level of sedation and the received EEG signal from the combined sensor 18. The combined sensor 18, that will be described in greater detail below, includes both a base strip 20 including multiple electrodes and an extension strip 22 that includes an acoustic emitter that allows the system of the present invention to carry out both active monitoring and passive monitoring by utilizing the same common sensor 18. As discussed above, in the preferred embodiment of the invention, the active monitoring module 14 can be the type of signal processing described in published PCT patent application WO 01/74248 while the passive monitoring module can be the type shown in published PCT patent application WO 02/32305. It should be understood that other active and passive systems could be utilized while operating within the scope of the present invention.

In the neuromonitoring system, the criteria for determining whether to utilize the active monitoring module or the passive monitoring module is determined by a minimum amplitude of the relevant AEP peak that can be reliably detected. When the amplitude of the AEP peak falls below a threshold value, which typically occurs when the level of sedation reaches Ramsay levels RS4 and RS5, the control unit 12 of the system 10 switches over to display the results on display 24 that are obtained by the passive module 16. When the relevant AEP peak is greater than the threshold value, the control unit 12 displays the results obtained by the active monitoring module 14.

Although the criteria for determining whether to utilize the active monitoring module or the passive monitoring module is described in the present invention as being related to the amplitude of the relevant AEP peak, it should be understood that various other criteria could be utilized while operating within the scope of the present invention. It is important that the control unit include software that allows the control unit to monitor some type of threshold value and, based upon the threshold value, select between the active monitoring module 14 and the passive monitoring module 16. The selection of the threshold value can vary depending upon the various active and passive monitoring modules, as well as the software contained within the control unit 12.

It should be understood that the neuromonitoring system 10 can utilize both the active and passive modes running simultaneously since these two modes do not interfere with each other. However, the control unit 12 typically displays only one of the two results to avoid complicating the already complicated intensive care data environment.

Typically, the passive monitoring module 16 is faster and is able to calculate a result from a time window of between five and fifteen seconds. The active monitoring module 14, on the other hand, can generate a sequence of AEP with 256 consecutive repetitions of impulses in approximately two minutes. However, the requirement for a fast response from the active monitoring module 14 is not necessary in monitoring an ICU patient over several days.

Referring now to Fig. 2, thereshown is a typical AEP signal 25 from a patient at a sample level of sedation. In the graph of Fig. 2, the peak of the signal is measured from the peak 26 (N_{b}). The latency of the signal is measured as the amount of time that has passed from applying the stimulus until the peak 26, as measured by time frame 28 in Fig. 2. In the example shown in Fig. 2, this latency period is approximately 45 milliseconds.

Although many methods of determining the amplitude of the peak can be used, one example is to measure the peak-to-peak difference between peak 26 (N_{b}) and peak 27 (P₁), as illustrated by amplitude 30. Typically, the value of the amplitude 30 is not critical, but rather the difference of the amplitude 30 from a baseline is the measured criteria.

Typically, the amplitude 30 and the latency 28 are patient-dependent such that absolute values for these two measurements cannot be used as threshold levels. Preferably, if a measurement is available at Ramsay level RS1, the threshold can be defined based on these relative values. For example, the control unit 12 can include an algorithm that is programmed to switch from the active monitoring module 14 to the passive monitoring module 16 when the amplitude 30 has dropped below 20% of its maximum value. If the measurement is used in typical ICU situations during the first days of care when the patient is emerging from deep sedation, the moment when the passive mode indicates the return of consciousness can be used as a criteria to move from the passive monitoring module to the active monitoring module. As discussed previously, the passive monitoring module is preferably used when monitoring a patient in Ramsay levels RS4 to RS6, while active monitoring module 14 is used when the patient is in Ramsay levels RS to RS3.

When a patient is in a level of sedation between Ramsay level RS3 and Ramsay level RS4, the control unit 12 utilizes software including fuzzy logic that takes into account both the information from the active monitoring module 14 and the passive monitoring module 16 to determine which monitoring mode to use on the display 24.

Although the system 10 shown in Fig. 1 utilizes both an active monitoring module 14 and a passive monitoring module 16, the system 10 is preferably utilized with a single sensor 18 developed in accordance with the present invention.

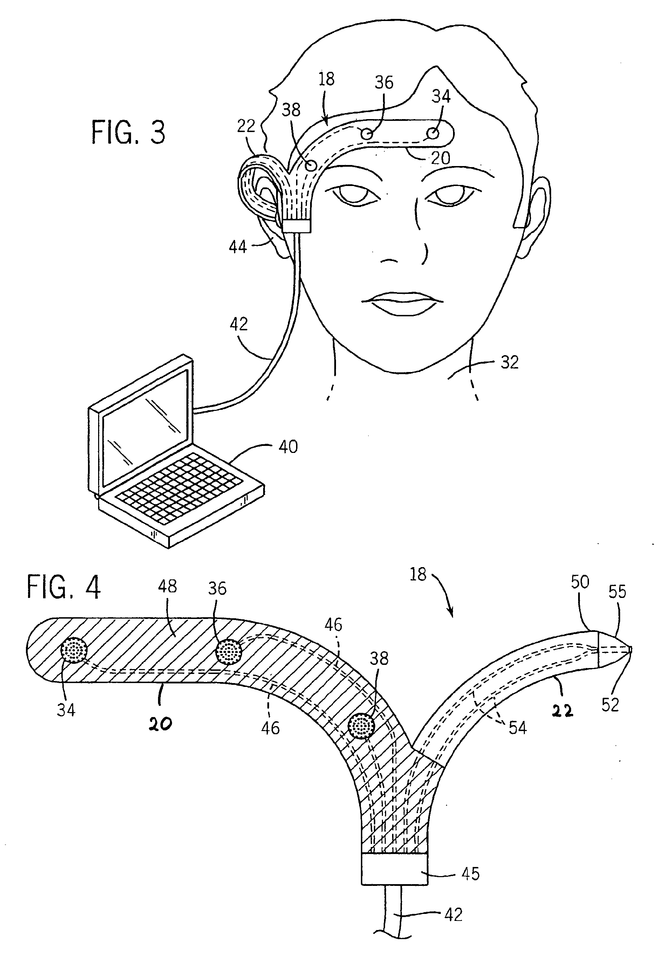

Referring now to Fig. 3, the sensor 18 of the present invention is shown applied to the forehead of a subject, such as patient 32. The sensor 18 has three individual electrodes 34, 36 and 38 that provide a single channel of EEG signal data to the control unit of the monitoring computer 40 through a single cable 42. Although a computer 40 is shown in Fig. 3, it should be understood that other types of processing components could be utilized to receive the data from cable 42.

In addition to being able to generate an EEG signal, the sensor 18 also functions as an AEP sensor such that the single sensor 18 can be utilized with both the active and passive monitoring modules.

Referring back to Fig. 3, the sensor 18 includes an extension strip 22 that extends from the base sensor strip 20 and is received within the patient's ear 44. The extension strip 22 is received within the patient's ear 44 to deliver auditory stimuli that are used by the active monitoring module 14 to induce the generation of an AEP signal that can then be sensed by the sensors 34, 36 and 38 placed on the patient's forehead.

Referring now to Fig. 4, thereshown is a detailed schematic illustration of the sensor 18 constructed in accordance with the present invention. As described previously, the sensor 18 includes a base sensor strip 20 and an extension strip 22. The base strip 20 and the extension strip 22 are formed as a unitary element of injected molded plastic material, such as polycarbonate plastic. The sensor 18 includes the common connector 45 connected to the cable 42.

As shown in Fig. 4, the base strip 20 includes the individual electrodes 34, 36, and 38, each of which include a lead 46 that extends to the connector 45. The leads 46 allow the individual electrodes to communicate to the control unit through the cable 42.

Each of the electrodes includes an outer surface that can be placed adjacent to the skin of the patient when the sensor and the plurality of electrodes are in use. In the embodiment shown in Fig. 4, there are three electrode areas 34, 36 and 38 to provide the three biopotential signals needed to form a single channel of the EEG signal data.

The portions of the base strip 20 not occupied by the electrode areas 34, 36 and 38 are formed to possess a desired degree of flexibility so as to allow the sensor 18 to conform to the contours of the skin of the patient on which the sensor 18 is placed. A variety of techniques may be used to render these portions of the base member flexible. For example, the portions of the base strip 20 may be sufficiently thin in a direction normal to the surface to render it flexible. For materials having the properties of polycarbonate plastic noted above, the thickness of the portions of the base strip 20 may be less than 0.5 millimeters, for example, 0.2 to 0.5 millimeters, or the portions of the base members may be perforated with holes to provide the desired amount of flexibility to the base strip 20.

The surface of the base strip 20 that will be adjacent to the skin of the patient may be provided with a coating of adhesive 48 to assist in retaining the sensor 18 on the skin of the patient. Preferably, adhesive layer 48 is provided on the portions of the base strip 20 not occupied by the sensors 34, 36 and 38.

It is not ordinarily necessary to use a wetting agent or conductive gel in conjunction with the sensor 18 since the base strip 20 can include microspikes to facilitate obtaining the biopotential signal. This feature, plus the ability to apply a plurality of electrodes at the same time, reduces the time needed to apply electrodes to the skin of the patient in the manner required to obtain the desired biopotential signal data.

The sensor 18 of the present invention includes the extension strip 22 that extends away from the base strip 20 but yet is connected to the connector 45. The extension strip 22 is formed of sufficient length to reach the ear 44 of a patient when the sensor 18 is applied to the patient as shown in Fig. 3. Referring back to Fig. 4, the extension strip 22 extends to an outer end 50 that includes an acoustic emitter 52. The acoustic emitter 52 is coupled to a pair of leads 54 that extend through the extension strip 22 and are joined to the cable 42 through the connector 45. The cable 42, in turn, is coupled to the control unit 12, such that the active monitoring module 14 can generate audible stimuli through the acoustic emitter 52.

In the preferred embodiment of the invention, the acoustic emitter 52 is surrounded by a soft, flexible plug 55 that can be fitted into the ear 44 of the patient 12. The soft plug 55 is designed such that the plug 55 stays in place in the ear channel of the patient for at least 24 hours. In accordance with the present invention, the entire sensor 18, including the extension strip 22 and acoustic emitter 52 are designed as a disposable product.

In traditional active acoustic monitoring methods, a significant amount of effort was placed in developing headphones or earphones that could be used to transmit the acoustic stimuli to the ear. Such high quality headphones or earphones are expensive and may not be practical in long-term, routine clinical use. Further, no solid evidence exists that the quality of the AEP signal can be improved by utilizing hi-fi compatible sound generators. The acoustical emitter 52 shown in Fig. 4 is a simple, miniature sound/click generator at the end of the extension strip 22 which is capable of reaching the ear channel of a patient.

Although the acoustic emitter 52 of the invention is shown in Fig. 4 as fitting within the ear of a patient, it should be understood that alternatively the acoustic emitter could be placed behind the back of the ear and transmit the acoustic stimuli to the patient through the bones of the skull behind the ear.

In the Figures of the present disclosure, the acoustic emitter 52 is shown placed in the right ear of the patient. However, it is possible that the patient may be hearing impaired in the right ear and not the left ear. Therefore, it is contemplated that an identical sensor could be designed such that the acoustic emitter 52 would be received in the left ear. The two mirror-image sensors could be stored in inventory and the correct sensor used when monitoring a patient that is hearing impaired in one ear and not the other.

In the preferred embodiment of the invention, the extension strip 22 is formed from the same material as the base strip 20 such that the extension strip and base strip can be formed as a single component.

Although the neuromonitoring system, not forming a part of the present invention, is shown in the Figures as including an integral sensor, according to the invention, including both the EEG electrodes and the acoustic emitter, it is contemplated that such a system could be operated utilizing a sensor for detecting EEG signals from the forehead of the patient that is separate from an acoustic emitter. In this alternate system, the control unit would still control the active and passive monitoring modules and would selectively display the level of sedation from only one of the active monitoring module or the passive monitoring module. In this alternate configuration, currently available sensors and acoustic emitter could be utilized.

Various alternatives and embodiments are contemplated as being within the scope of the following claims particularly pointing out and distinctly claiming the subject matter regarded as the invention.

## Claims

1. A unitary sensor for detecting biopotential signals on the skin of a patient, the sensor comprising:
a base strip (20) including at least three electrodes (34, 36, 38) spaced along the length of the base strip, each of the sensors being operable to detect biopotential signals on the skin of the patient; and **characterised by**
an extension strip (22) integrally formed with the base strip and extending away from the base strip to a distal end (50), the extension strip including an acoustic emitter (52) attached to the distal end (50), and the base strip (20) being configured to be attached to the forehead of the patient and the extension strip (22) extends from the base strip such that the acoustic emitter (52) is positionable in an ear of the patient when the base strip is positioned on the forehead of the patient.

2. The unitary sensor of claim 1 wherein the acoustic emitter (52) is surrounded by a resilient plug (55) such that the plug can be placed within the ear of the patient.

3. The unitary sensor of claim 1 or 2 wherein each of the plurality of sensors is operable to detect both EEG signals and AEP signals.

4. The unitary sensor of any preceding claim wherein the base strip (20) includes an adhesive to secure the base strip to the patient.

5. The unitary sensor of any preceding claim further comprising a connector portion coupled to both the base strip (20) and the extension strip (22), the connector portion receiving a lead from each of the plurality of sensors and a pair of leads from the acoustic emitter (52).

6. The unitary sensor of any preceding claim wherein the acoustic emitter is operable to emit an audible stimulus.

## Patentansprüche

1. Unitärer Sensor für die Erfassung von Biopotenzialsignalen auf der Haut eines Patienten, wobei der Sensor umfasst:
einen Basisstreifen (20), enthaltend mindestens drei Elektroden (34, 36, 38), welche entlang der Länge des Basisstreifens voneinander beabstandet angeordnet sind, wobei jeder einzelne der Sensoren funktionsfähig ist, um Biopotenzialsignale auf der Haut des Patienten zu erfassen; und **dadurch gekennzeichnet ist, dass**
ein Verlängerungsstreifen (22), der integral mit dem Basisstreifen gebildet ist, sich von dem Basisstreifen weg zu einem distalen Ende (50) erstreckt, wobei der Verlängerungsstreifen einen akustischen Emitter (52) umfasst, welcher an dem distalen Ende (50) befestigt ist, und der Basisstreifen (20) so ausgeführt ist, dass er an der Stirn des Patienten befestigt werden kann, und der Verlängerungsstreifen (22) sich von dem Basisstreifen so erstreckt, dass der akustische Emitter (52) in einem Ohr des Patienten platziert werden kann, wenn der Basisstreifen auf der Stirn des Patienten positioniert ist.

2. Unitärer Sensor nach Anspruch 1, wobei der akustische Emitter (52) so von einem elastischen Stopfen (55) umgeben ist, dass der Stopfen in dem Ohr des Patienten platziert werden kann.

3. Unitärer Sensor nach Anspruch 1 oder 2, wobei jeder einzelne Sensor aus der Vielzahl von Sensoren funktionsfähig ist, um sowohl EEG-Signale als auch AFP-Signale zu erfassen.

4. Unitärer Sensor nach einem der vorhergehenden Anspruche, wobei der Basisstreifen (20) einen Klebstoff enthält, um den Basisstreifen am Patienten zu befestigen.

5. Unitärer Sensor nach einem der vorhergehenden Ansprüche, ferner umfassend einen Verbindungsabschnitt, welcher sowohl mit dem Basisstreifen (20) als auch mit dem Verlängerungsstreifen (22) verbunden ist, wobei der Verbindungsabschnitt eine Ableitung von jedem einzelnen Sensor aus der Vielzahl von Sensoren erhält sowie zwei Ableitungen von dem akustischen Emitter (52).

6. Unitärer Sensor nach einem der vorhergehenden Ansprüche, wobei der akustische Emitter funktionsfähig ist, um einen hörbaren Reiz auszusenden.

## Revendications

1. Ensemble capteur destiné à la détection de signaux biopotentiels sur la peau d'un patient, le capteur comprenant :
une bande de base (20) comprenant au moins trois électrodes (34, 36, 38) espacées le long de la longueur de la bande de base, chacun des capteurs étant en opérabilité pour détecter des signaux biopotentiels sur la peau du patient ; et **caractérisé par**
une bande d'extension (22) intégralement formée avec la bande de base et s'étendant à distance de la bande de base jusqu'à une extrémité distale (50), la bande d'extension comprenant un émetteur acoustique (52) fixé à l'extrémité distale (50) et la bande de base (20) étant configurée pour être attachée au front du patient et la bande d'extension (22) s'étendant de la bande de base de manière à ce que l'émetteur acoustique (52) soit positionnable dans une oreille du patient lorsque la bande de base est positionnée sur le front du patient.

2. Ensemble capteur de la revendication 1 dans lequel l'émetteur acoustique (52) est entouré d'une fiche à pression (55) de manière à ce que la fiche puisse être placée dans l'oreille du patient.

3. Ensemble capteur de la revendication 1 ou 2, dans laquelle chacun de la pluralité des capteurs est en opérabilité pour détecter à la fois les signaux EEG et AEP.

4. Ensemble capteur de l'une quelconque des revendications précédentes, dans lequel la bande de base (20) contient un adhésif pour fixer la bande de base au patient.

5. Ensemble capteur de l'une quelconque des revendications précédentes comprenant en outre une partie de connecteur couplée à la fois à la bande de base (20) et à la bande d'extension (22), la partie de connecteur recevant un cordon de chacun de la pluralité des capteurs et une paire de cordons de l'émetteur acoustique (52).

6. Ensemble capteur de l'une quelconque des revendications précédentes, dans lequel l'émetteur acoustique est en opérabilité pour émettre un stimulus sonore.
